(19) Europäisches Patentamt / European Patent Office / Office européen des brevets

(11) EP 3 689 858 A1

(12) **EUROPEAN PATENT APPLICATION**
published in accordance with Art. 153(4) EPC

(43) Date of publication:
**05.08.2020 Bulletin 2020/32**

(21) Application number: **18863230.1**

(22) Date of filing: **27.09.2018**

(51) Int Cl.:
***C07D 309/10*** *(2006.01)* ***A61K 31/351*** *(2006.01)*

(86) International application number:
**PCT/KR2018/011383**

(87) International publication number:
**WO 2019/066467 (04.04.2019 Gazette 2019/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.09.2017 KR 20170125928**

(71) Applicant: **Hanmi Pharm. Co., Ltd.**
**Hwaseong-si, Gyeonggi-do 18536 (KR)**

(72) Inventors:
- **JANG, Wook**
  **Hwaseong-si**
  **Gyeonggi-do 18469 (KR)**
- **BAEK, Jong Ouk**
  **Hwaseong-si**
  **Gyeonggi-do 18469 (KR)**
- **KIM, Hee Cheol**
  **Hwaseong-si**
  **Gyeonggi-do 18469 (KR)**
- **HA, Tae Hee**
  **Hwaseong-si**
  **Gyeonggi-do 18469 (KR)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) NOVEL METHOD FOR PREPARING (2R)-2-(2-METHOXYPHENYL)-2-(OXANE-4-YLOXY)ETHANE-1-OL COMPOUND, AND INTERMEDIATE USED THEREIN

(57) Provided in the present invention are: a novel method for preparing a compound of Chemical Formula 1, comprising a step of obtaining a compound of Chemical Formula 1 by performing a reduction reaction on a compound of Chemical Formula 2; and an intermediate used therein. According to the preparation method of the present invention, the compound of Chemical Formula 1, which is useful for the production of an acetyl-CoA carboxylase (ACC) inhibitor, can be mass-produced more simply and efficiently than that of a conventional method.

EP 3 689 858 A1

## Description

[Technical Field]

The present invention relates to useful compounds that show activity as inhibitors of acetyl-CoA carboxylase (ACC), in particular, to a novel method for preparing the compound (2R)-2-(2-methoxyphenyl)-2-(oxan-4-yloxy)ethan-1-ol which is a structure commonly included in the structure of thienopyrimidine derivative, thienothiadiazine derivative, thienopyridazine derivative and quinazoline derivative compounds, and novel intermediates used therein.

[Background Art]

Inhibitors of acetyl-CoA carboxylase (ACC) have been shown to be effective in the treatment of ACC-mediated disorders such as obesity, dyslipidemia, hyperlipidemia, fungal infections, parasitic infections or bacterial infections, and thus a great deal of research has been done on it.

PCT publications WO 2013/071169, WO 2014/182950, and WO 2014/182951 disclose the structures of compounds useful as an inhibitor of acetyl-CoA carboxylase (ACC), in particular thienopyrimidine derivative, thienothiadiazine derivative, thienopyridazine derivative and quinazoline derivative compounds, and the structures of these compounds showing good activity commonly include a structure of (2R)-2-(2-methoxyphenyl)-2-(oxan-4-yloxy)ethan-1-ol, a compound of Formula 1.

[Formula 1]

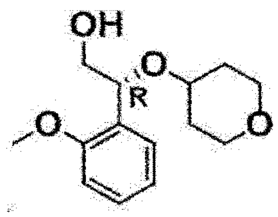

In addition, the document WO 2013/071169 discloses a method for preparing the compound of Formula 1. Specifically, as shown in Reaction Scheme 1 below, 2-methoxybenzaldehyde of Formula A is reacted with S,S-dimethylmethanesulfinyl iodide (trimethylsulfoxonium iodide) under sodium hydride (NaH) to prepare the compound of Formula B, and the compound of Formula B is reacted with oxan-4-ol under iron (III) chloride ($FeCl_3$) to produce the compound of Formula C. Thereafter, the compound of Formula 1 is prepared from the compound of Formula C using chiral preparative high-performance liquid chromatography equipment.

[Reaction Scheme 1]

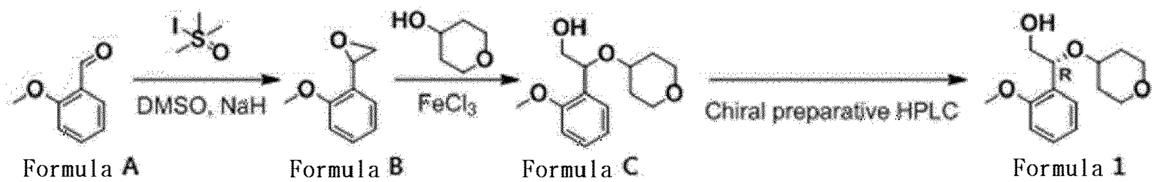


Formula A Formula B Formula C Formula 1

However, the synthesis method through the route of Reaction Scheme 1 has the following problems in the manufacturing method. In the step of obtaining the compound of Formula B from the compound of Formula A, an unfamiliar reagent such as S,S-dimethylmethanesulfinyl iodide is used and sodium hydride (NaH), a flammable substance, is used. In addition, the yield in the step of obtaining the compound of Formula C is as low as 21% and there is a difficulty in that the generated impurities have to be purified using column chromatography.

In addition, this method can be said to be unsuitable for industrial mass production because it must be obtained by mechanical separation using chiral preparative high-performance liquid chromatography equipment in the separation step of the final optical isomer to obtain the compound of Formula 1 by the route of Reaction Scheme 1.

[Prior Art Document]

(Patent Document 1) WO 2013/071169
(Patent Document 2) WO 2014/182950
(Patent Document 3) WO 2014/182951

[Disclosure]

[Technical Problem]

The present invention has been made to solve the above problems of the prior art, and it is an object of the present invention to provide a method for mass-producing (2R)-2-(2-methoxyphenyl)-2-(oxan-4-yloxy)ethan-1-ol, a compound of Formula 1, simply and efficiently.

It is also an object of the present invention to provide novel intermediate compounds used in the preparation of (2R)-2-(2-methoxyphenyl)-2-(oxan-4-yloxy)ethan-1-ol, the compound of Formula 1.

[Technical Solution]

In order to achieve the above objects, the present invention provides a method for preparing the compound of Formula 1 below comprising a step of performing a reduction reaction on the compound of Formula 2 below to obtain the compound of Formula 1:

[Formula 1]

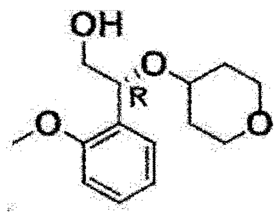

[Formula 2]

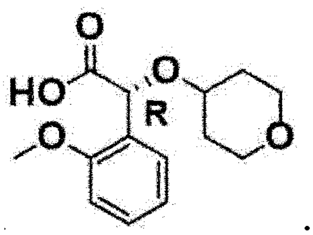

The compound of Formula 2 may be prepared by comprising a step of neutralizing the compound of Formula 3 below in the presence of an acid, followed by extracting to obtain the compound of Formula 2 from an organic layer:

[Formula 3]

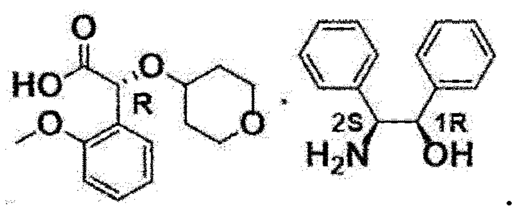

The compound of Formula 3 may be prepared by comprising a step of producing a salt of the compound of Formula 4 below and the compound of Formula 8 below:

[Formula 4]

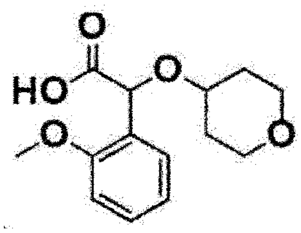

[Formula 8]

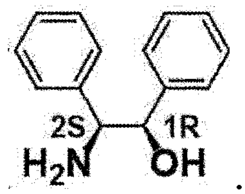

The compound of Formula 4 may be prepared by comprising a step of hydrolyzing the compound of Formula 5 below in the presence of a base:

[Formula 5]

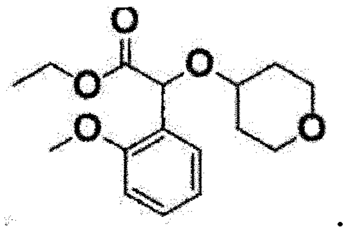

The compound of Formula 5 may be prepared by comprising a step of introducing the leaving group to the compound of Formula 6 below and performing a substitution reaction with the compound of Formula 7 below:

[Formula 6]

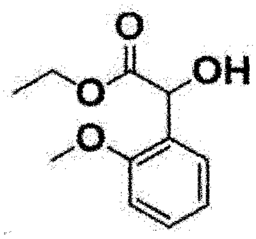

[Formula 7]

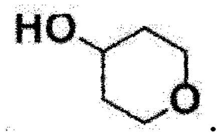

The present invention also provides a compound represented by Formula 2 below which is an intermediate of the compound Formula 1:

[Formula 2]

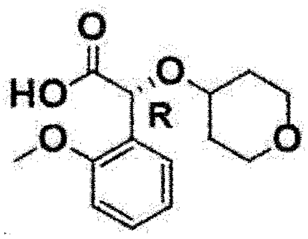

.

The present invention also provides a compound represented by Formula 3 below which is an intermediate of the compound Formula 1:

[Formula 3]

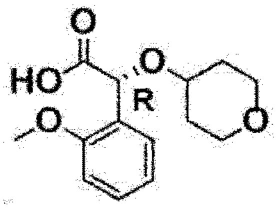

·

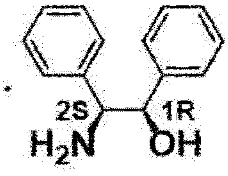

.

The present invention also provides a compound represented by Formula 4 below which is an intermediate of the compound of Formula 1:

[Formula 4]

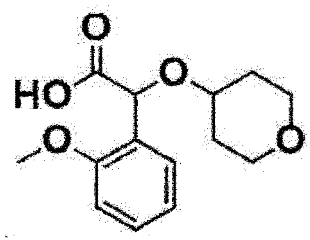

.

The present invention also provides a compound represented by Formula 5 below which is an intermediate of the compound of Formula 1:

[Formula 5]

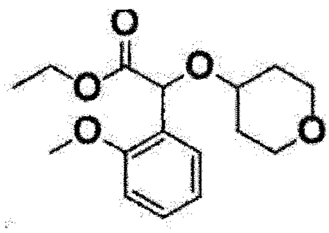

.

[Advantageous Effects]

According to the preparation method of the present invention, it is possible to mass-produce (2R)-2-(2-methoxyphenyl)-2-(oxan-4-yloxy)ethan-1-ol, the compound of Formula 1 in a very simple and efficient manner, which is

commonly included in compounds useful as an inhibitor of acetyl-CoA carboxylase (ACC).

[Best Mode]

Hereinafter, the present invention will be described in detail.

The present invention relates to a method for preparing the compound of Formula 1 below comprising a step of performing the reduction reaction on the compound of Formula 2 below to obtain the compound of Formula 1:

[Formula 1]

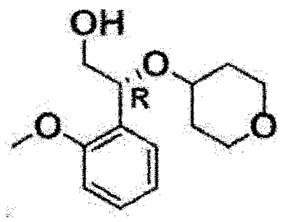

[Formula 2]

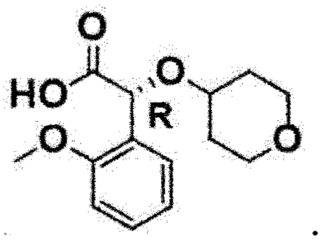

The reduction reaction is carried out using at least one selected from the group consisting of lithium aluminum hydride ($LiAlH_4$, LAH), borane dimethyl sulfide ($BH_3S(CH_3)_2$, BMS), lithium borohydride ($LiBH_4$), aluminum hydride ($AlH_3$), and RED-AL (Vitride) as a reducing agent. Preferably, lithium aluminum hydride ($LiAlH_4$, LAH) or borane dimethyl sulfide ($BH_3S(CH_3)_2$, BMS) may be used.

The reducing agent may be used in an amount of 1.0 mole equivalent to 3.0 mole equivalents, specifically 1.5 mole equivalents to 2.0 mole equivalents, relative to 1 mole equivalent of the compound of Formula 2.

The reduction reaction may be performed in a solution, and the solvent constituting the solution may be tetrahydrofuran, dioxane, diethyl ether, toluene, N',N'-dimethylformamide, N',N-dimethylacetamide, dimethyl sulfoxide, or mixtures thereof. Preferably, tetrahydrofuran, diethyl ether or the like may be used. The amount of organic solvent used may be 5 ml to 20 ml, specifically 10 ml to 15 ml, relative to 1 g of the compound of Formula 2.

The reaction may be performed at 0°C to 30°C, specifically 10°C to 20°C, for 2 hours to 24 hours, specifically 6 hours to 12 hours.

In the preparation method of the present invention, the compound of Formula 2 may be prepared by comprising a step of neutralizing the compound of Formula 3 below in the presence of an acid, and then extracting to obtain the compound of Formula 2 from an organic layer.

[Formula 3]

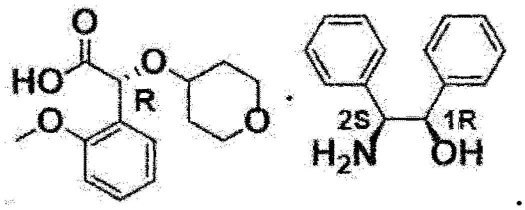

Neutralization of the compound of Formula 3 can be carried out using at least one acid selected from the group consisting of inorganic and organic acids.

The inorganic acid may be hydrochloric acid, nitric acid, sulfuric acid, and the like, and the organic acid may

be acetic acid, trifluoroacetic acid, or the like. Preferably acetic acid or hydrochloric acid may be used.

The acidity (pH) suitable for the neutralization extraction is preferably 0.1 to 4.0, specifically 1.0 to 2.0.

The solvent used for the extraction may be any one selected from the group consisting of dichloromethane, methyl acetate, ethyl acetate, isopropyl acetate, diethyl ether, and mixtures thereof. Preferably dichloromethane or ethyl acetate may be used.

In the preparation method of the present invention, the compound of Formula 3 may be prepared by comprising a step of producing a salt from the compound of Formula 4 below and the compound of Formula 8 below.

[Formula 4]

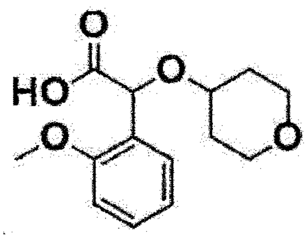

[Formula 8]

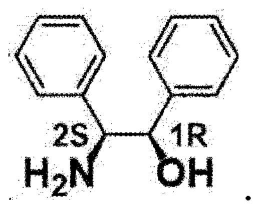

The salt may be formed in a solution, and the solvent constituting the solution may be at least one selected from the group consisting of methanol, ethanol, isopropanol, acetonitrile, toluene, acetone, methyl ethyl ketone, methyl isobutyl ketone, methyl acetate, ethyl acetate, isopropyl acetate, diethyl ether and the like. Preferably ethyl acetate, isopropyl acetate and the like can be used.

The organic solvent may be used in combination with water, wherein the mixing ratio of the organic solvent and water may vary depending on the type of organic organic solvent. If ethyl acetate or isopropyl acetate is used as an organic solvent, the mixing ratio of the organic solvent and water may be 9:1 to 9.9:0.1 in volume ratio. Specifically, water concentration in a mixed solvent may be 1% (v/v) to 10% (v/v).

The reaction may be performed under 0°C to 30°C, specifically 10°C to 20°C, for 2 hours to 24 hours, specifically 6 hours to 12 hours.

The present invention may comprise a seeding process in order to efficiently produce the salt (the compound of Formula 3) from the compound of Formula 4 and the compound of Formula 8, and may further comprise a step of increasing the purity of the compound of Formula 3 obtained by the above method. The step of increasing the purity is a step of further purifying the compound of Formula 3 after producing the salt (the compound of Formula 3) from the compound of Formula 4 and the compound of Formula 8, which may be carried out in a manner of adding a mixed solvent of an organic solvent and water to the compound of Formula 3, warming to the reflux temperature of the mixed solvent while stirring, and cooling it to room temperature and then filtering the reprecipitated solid.

The recrystallization method for obtaining the compound of Formula 3 having high purity and optical activity may be performed for 1 hour to 2 hours after heating at 40°C to 80°C , specifically 60°C to 70°C, and then for 2 hours to 12 hours after cooling to 0°C to 30°C, specifically 10°C to 20°C, and the same method can be repeated several times.

In the preparation method of the present invention, the compound of Formula 4 may be prepared by comprising a step of hydrolyzing the compound of Formula 5 below in the presence of a base.

[Formula 5]

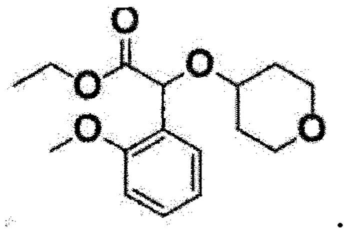

The hydrolysis may be performed using at least one base selected from the group consisting of potassium carbonate, calcium carbonate, sodium carbonate, potassium hydroxide, sodium hydroxide, lithium hydroxide, sodium hydrogen carbonate, and the like, and preferably sodium hydroxide, potassium hydroxide, and lithium hydroxide can be used.

The base used in the reaction may be used in an amount of 1.0 mole equivalent to 5.0 mole equivalents, specifically 2.0 mole equivalents to 3.0 mole equivalents, relative to 1 mole equivalent of the compound of Formula 5.

The reaction may be carried out under 10 °C to 30 °C, specifically 15 °C to 25 °C, for 1 hour to 5 hours, specifically 2 hours to 3 hours.

The hydrolysis reaction may be carried out in a solution, and the solvent constituting the solution may be acetone, methanol, ethanol, isopropanol, acetonitrile and mixtures thereof. Preferably the solvent may be methanol or ethanol.

The organic solvent may be used in combination with water, wherein the mixing ratio of the organic solvent and water may vary depending on the type of solvent. If methanol or ethanol is used as an organic solvent, the mixing ratio of the organic solvent and water may be 9:1 to 1:9 by volume. Specifically, the mixing ratio of the organic solvent and water may be 4:1 to 1:1.

In the preparation method of the present invention, the compound of Formula 5 may be prepared by comprising a step of introducing the leaving group to the compound of Formula 6 below and performing a substitution reaction with the compound of Formula 7 below.

[Formula 6]

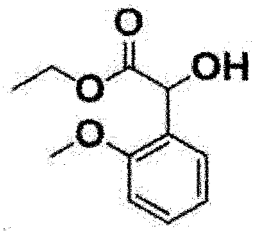

[Formula 7]

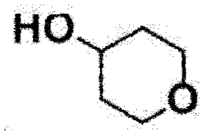

The leaving group may be introduced by reacting the leaving group reagent in the reaction solution. The leaving group reagent may be selected from the group consisting of sulfonating agents such as methanesulfonyl chloride, toluenesulfonyl chloride, and benzenesulfonyl chloride, and halogenating agent such as chlorine (C12), bromine (Br2), iodide (I2), oxalyl chloride ((COCl)2), thionyl chloride (SOCl2) and sulfuryl chloride (S02C12), and mixtures thereof. Preferably, methanesulfonyl chloride and toluenesulfonyl chloride can be used.

The leaving group reagent may be used in an amount of 1.0 mole equivalent to 2.0 mole equivalents, specifically 1.2 mole equivalents to 1.5 mole equivalents, relative to 1 mole equivalent of the compound of Formula 6.

Specifically, the leaving group may be methanesulfonyl, toluenesulfonyl, benzenesulfonyl, halogen (Br, Cl, I) and the like.

The introduction of the leaving group may be performed at 0°C to 40°C, specifically 15°C to 25°C, for 0.5 hours

to 3 hours, specifically 1 hour to 2 hours.

The leaving group introduction reaction may be performed in the presence of a base in the solution. The solvent included in the solution may be selected from the group consisting of tetrahydrofuran, dioxane, dichloromethane, chloroform, acetonitrile, toluene, acetone, methyl ethyl ketone, methyl isobutyl ketone, methyl acetate, ethyl acetate, isopropyl acetate, N',N'-dimethylformamide, N',N-dimethylacetamide, dimethyl sulfoxide and mixtures thereof. Preferably, dichloromethane and acetonitrile can be used. The amount of the solvent used may be 5 ml to 20 ml, specifically 10 ml to 15 ml, relative to 1 g of the compound of Formula 6.

In addition, the base used in the reaction may be selected from the group consisting of triethylamine, trimethylamine, diisopropylethylamine, pyridine, tetramethylethylenediamine and the like. Preferably, triethylamine or diisopropylethylamine may be used. The base used in the reaction may be used in an amount of 1.0 to 5.0 mole equivalents, specifically 2.0 to 3.0 mole equivalents, relative to 1 mole equivalent of the compound of Formula 6.

In the step of reacting with the compound of Formula 7 after introduction of the leaving group, the compound of Formula 7 may be used in an amount of 1.0 to 1.5 mole equivalents, specifically 1.2 to 1.3 mole equivalents, relative to 1 mole equivalent of the compound of Formula 6.

In addition, the solvent used in the reaction may be any one selected from the group consisting of dichloromethane, chloroform, acetonitrile, toluene, acetone, methyl ethyl ketone, methyl isobutyl ketone, methyl acetate, ethyl acetate, isopropyl acetate, N',N'-dimethylformamide, N',N-dimethylacetamide, dimethyl sulfoxide and mixtures thereof. Specifically, acetonitrile or toluene may be preferably used.

The reaction may be performed at 60°C to 120°C, specifically 80°C to 110°C, for 6 hours to 24 hours, specifically 12 hours to 16 hours.

The compound of Formula 6, the compound of Formula 7 and the compound of Formula 8 used for optical splitting, which are used as starting materials in the aforementioned method, can be purchased commercially or prepared according to conventional methods.

The method for preparing the compound of Formula 1 of the present invention is represented by a series of reaction schemes as follows:

[Reaction Scheme 2]

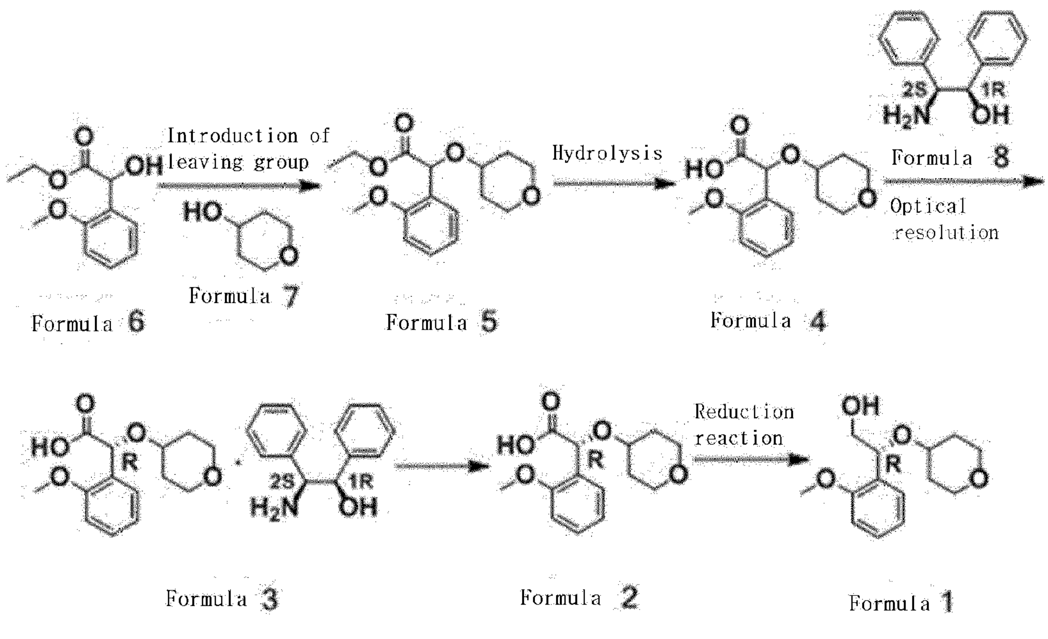


The present invention also provides a compound represented by Formula 2 below which is an intermediate of the compound of Formula 1:

[Formula 2]

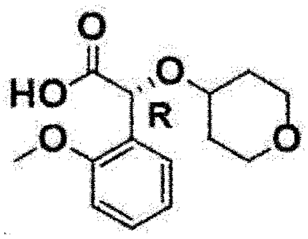

.

The present invention also provides a compound represented by Formula 3 below which is an intermediate of the compound of Formula 1:

[Formula 3]

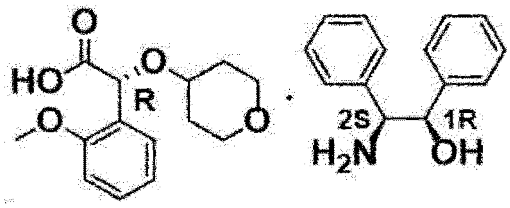

,

The present invention also provides a compound represented by Formula 4 below which is an intermediate of the compound of Formula 1:

[Formula 4]

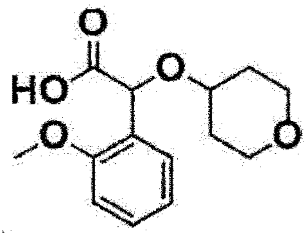

.

The present invention also provides a compound represented by Formula 5 below which is an intermediate of the compound of Formula 1:

[Formula 5]

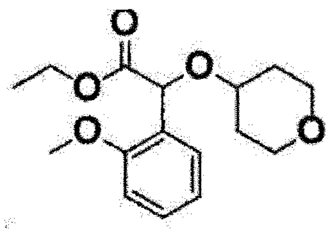

.

The intermediates are novel compounds and may be useful for the preparation of compound of Formula 1.

Hereinafter, the present invention will be described in more detail with reference to examples. However, the following examples are provided to more specifically describe the present invention, and the scope of the present invention is not limited to the following examples. The following examples can be appropriately modified and changed

by those skilled in the art within the scope of the present invention.

<Calculation method of optical purity>

The optical purities described in examples are values calculated from the areas shown in the chromatogram after separating each isomer according to the following conditions.

1) Analysis condition

(1) Analysis conditions for optical purities of Formula 2 and Formula 3

Detector: UV spectrophotometer (detection wavelength: 205 nm)
Column: Chiralcel OD-RH (4.6 x 150mm)
Mobile phase: Mobile phase A / Mobile phase B = 80 / 20
Mobile phase A: pH 2.5 buffer [$NaClO_4$ 7g+$KH_2PO_4$ 1.7g/L pH adjusted $H_3PO_4$]
Mobile phase B: 100% ACN
Flow rate: 0.9 ml/min.
Temperature: 35°C

(2) Analysis conditions for optical purity of Formula 1

Detector: UV spectrophotometer (detection wavelength: 205 nm)
Column: Chiralcel OD-RH (4.6 x 150mm)
Mobile phase: Mobile phase A / Mobile phase B = 80 / 20
Mobile phase A: pH 2.5 buffer [$NaClO_4$ 7g+$KH_2PO_4$ 1.7g/L pH adjusted $H_3PO_4$]
Mobile phase B: 100% ACN
Flow rate: 0.8 ml/min.
Temperature: 30°C

2) Calculation formula

$$\text{Optical purity (\%)} = PR / (PS + PR) \times 100$$

wherein PR is the peak area of the corresponding (R)-isomer obtained in the chromatogram and PS is the peak area of the (S)-isomer obtained in the chromatogram.

**<Preparation of (2R)-2-(2-methoxyphenyl)-2-(oxan-4-yloxy)ethan-1-ol (Formula 1)>**

**Example 1: Preparation of ethyl 2-(2-methoxyphenyl)-2-((tetrahydro-2H-pyran-4-yl)oxy)acetate (Formula 5)**

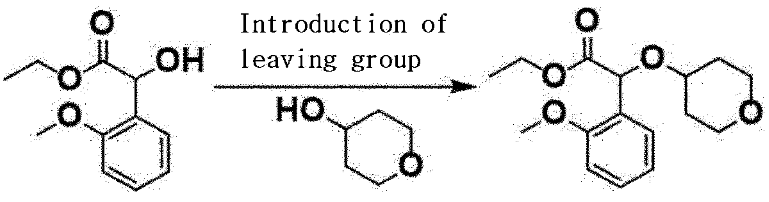


Formula 7

Formula 6 Formula 5

90 g (0.428 mol) of ethyl 2-hydroxy-2-(2-methoxyphenyl) acetate of Formula 6 was dissolved in 900 ml of dichloromethane, and then 119 ml (0.856 mol) of triethylamine was added. After cooling the reaction temperature to 10 °C or less, 39.8 ml (0.514 mol) of methane sulfonyl chloride was slowly added while maintaining the temperature at 10 °C or less, and the reaction temperature was raised to room temperature, followed by stirring for 1 hour. After the reaction was completed, the mixture was washed with 900 ml of water, dried over anhydrous magnesium sulfate and distilled under reduced pressure to obtain 120 g (yield 97%) of ethyl 2-(2-methoxyphenyl)-2-((methylsulfonyl)oxy)acetate compound as an oil phase.

1H NMR (300 MHz, CDC13): δ7.43-7.33 (m, 2H), 7.01-6.93 (m, 2H), 6.31 (s, 1H), 4.26 (q, 2H), 3.87 (s, 3H), 3.09 (s, 3H), 1.24 (t, 3H).

120 g (0.416 mol) of ethyl 2-(2-methoxyphenyl)-2-((methylsulfonyl)oxy)acetate was dissolved in 900 ml of toluene and 149 ml (0.856 mol) of diisopropylethylamine was added thereto. 49 ml (0.514 mol) of tetrahydro-2H-pyran-4-ol of Formula 7 was added thereto, followed by heating to reflux for 16 hours at 100°C to 110°C. After the reaction was completed, 900 ml of ethyl acetate and 900 ml of water were added, and the organic layer was separated. The obtained organic layer was washed with 450 ml of aqueous saturated sodium chloride solution, dried over anhydrous magnesium sulfate, and distilled under reduced pressure to obtain 85 g (yield 70%) of the titled compound (Formula 5) as an oil phase.

1H NMR (300 MHz, CDC13): δ7.45-7.42 (m, 1H), 7.32-7.27 (m, 1H), 7.09-6.89 (m, 2H), 5.45 (s, 1H), 4.17 (q, 2H), 3.98-3.84 (m, 2H), 3.83 (s, 3H), 3.66-3.61 (m, 1H), 3.43-3.38 (m, 2H), 2.04-1.72 (m, 2H), 1.71-1.62 (m, 2H), 1.22 (t, 3H).

**Example 2: Preparation of 2-(2-methoxyphenyl)-2-((tetrahydro-2H-pyran-4-yl)oxy)acetic acid (Formula 4)**

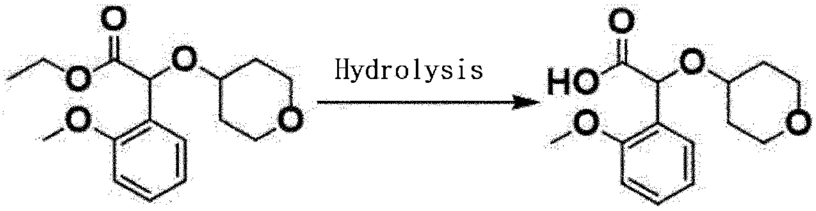


Formula 5 Formula 4

80 g (0.271 mol) of ethyl 2-(2-methoxyphenyl)-2-((tetrahydro-2H-pyran-4-yl)oxy)acetate of Formula 5 prepared in Example 1 was dissolved in 640 ml of ethanol, and an aqueous solution of 22.8 g (0.543 mol) of lithium hydroxide dissolved in 160 ml of water was added thereto, followed by stirring at room temperature for 2 hours. After the reaction was completed, 800 ml of water and 1,200 ml of ethyl acetate were added sequentially and separated to remove the organic layer. The aqueous layer was washed once more with 400 ml of ethyl acetate and the pH of the aqueous layer was adjusted to pH 1 to 2 using hydrochloric acid and extracted twice with 800 ml and 400 ml of dichloromethane. The product was dried over anhydrous magnesium sulfate and distilled under reduced pressure to obtain 70 g (yield 97%) of the titled compound (Formula 4) as a foam phase.

1H-NMR (300 MHz, CDC13): 7.40-7.31 (m, 2H), 7.00-6.90 (m, 2H), 5.43 (s, 1H), 3.98-3.88 (m, 2H), 3.85 (s, 3H), 3.64-3.61 (m, 1H), 3.42-3.37 (m, 2H), 1.78-1.68 (m, 2H), 1.68-1.63 (m, 2H).

**Example 3: Preparation of salt (Formula 3) from 2-(2-methoxyphenyl)-2-((tetrahydro-2H-pyran-4-yl)oxy)acetic acid (Formula 4) and (1R,2S)-2-amino-1,2-diphenylethanol(Formula 8)**

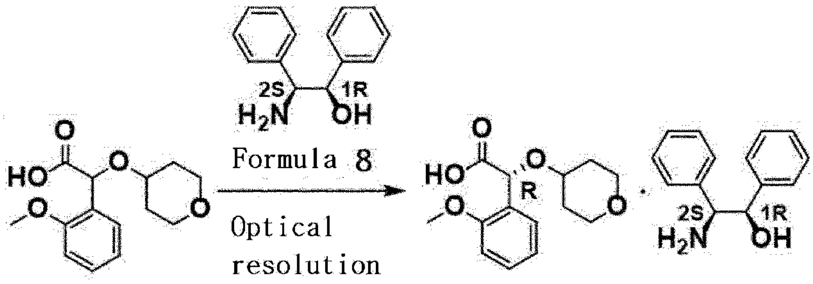


Formula 4 Formula 3

After dissolving 70 g (0.262 mol) of 2-(2-methoxyphenyl)-2-((tetrahydro-2H-pyran-4-yl)oxy)acetic acid of Formula 4 prepared in Example 2 in 490 ml of ethyl acetate, 56 g (0.262 mol) of (1R,2S)-2-amino-1,2-diphenylethanol of Formula 8 was added thereto, followed by stirring at room temperature for 20 hours. The resulting solid compound was filtered, washed with 70 ml of ethyl acetate and dried to obtain 71 g (yield 56%, HPLC optical purity 65.2%) of the crude product of the titled compound (Formula 3) as a solid phase.

70 g (0.146 mol) of the crude product of the titled compound (Formula 3) was added to 490 ml of ethyl acetate containing water and heated to reflux at 60°C to 70°C for 30 minutes. The reaction solution was cooled to room temperature and stirred for 16 hours. The resulting solid was filtered, washed with 70 ml of ethyl acetate and dried to obtain 28.8 g (yield 40%, HPLC optical purity 91%) of the titled compound (Formula 3) having high optical purity. The same method was applied two to three times to obtain 20 g (yield 70%, HPLC optical purity> 98%) of the titled compound (Formula 3) of high optical purity.
Melting point: 163°C~166°C
1H NMR (300 MHz, DMSO-d6): 7.37-7.33 (m, 2H), 7.26-7.10 (m, 10H), 6.99-6.91 (m, 2H), 5.22 (s, 1H), 4.92 (d, 1H), 4.19 (d, 1H), 3.81-3.74 (m, 5H), 3.58-3.56 (m, 1H), 3.32-3.24 (m, 2H), 1.84-1.78 (m, 2H), 1.47-1.36 (m, 2H).

**Example 4: Preparation of (R)-2-(2-methoxyphenyl)-2-((tetrahydro-2H-pyran-4-yl)oxy)acetic acid (Formula 2)**

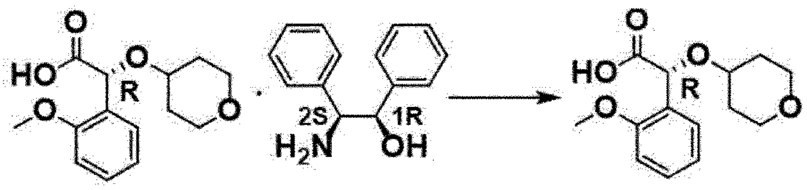

Formula 3 Formula 2

19 g (0.040 mol) of the compound of Formula 3 prepared in Example 3 were added to 190 ml of dichloromethane and 190 ml of water, the pH was adjusted to 1 to 2 using 1N aqueous hydrochloric acid solution, the organic layer was taken, and the aqueous layer was extracted once more using 190 ml of dichloromethane. The organic layer was dried over anhydrous magnesium sulfate and distilled under reduced pressure to obtain 8.8 g (84%) of the titled compound (Formula 3).
Melting point: 120°C~125°C
GC/MS spectrum: m/z=265.02 (M-1)

The pH of the aqueous layer was adjusted to pH 7 to pH 8 using aqueous sodium hydroxide solution, and the resulting solid was filtered, washed with water and dried to obtain 7.5 g (94%) of (1R,2S)-2-amino-1,2-diphenylethanol (Formula 8) .

**Example 5: Preparation of (R)-2-(2-methoxyphenyl)-2-((tetrahydro-2H-pyran-4-yl)oxy)ethan-1-ol (Formula 1)**

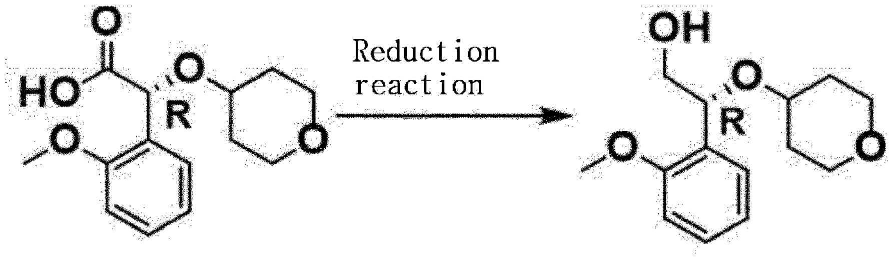


Formula 2 Formula 1

**Example 5.1: Reduction reaction using lithium aluminum hydride ($LiAlH_4$)**

7.7 g (0.029 mol) of (R)-2-(2-methoxyphenyl)-2-((tetrahydro-2H-pyran-4-yl)oxy)acetic acid of Formula 2 prepared in Example 4 was added to 115 ml of tetrahydrofuran. Thereafter, the internal temperature of the reaction solution was cooled to 5 °C or less, and 1.3 g (0.035 mol) of lithium aluminum hydride was slowly added. After stirring for 1 hour while maintaining the reaction temperature at 5 °C or less, additionally 0.23 g (0.006 mol) of lithium aluminum hydride was slowly added and stirred for 1 hour. After the reaction was completed, aqueous saturated ammonium chloride solution was added and extracted three times using 115 ml of ethyl acetate respectively. The organic layer was dried over anhydrous magnesium sulfate, distilled under reduced pressure and purified by column chromatography to obtain 6.3 g (85%) of the titled compound (Formula 1) as a solid phase.

**Example 5.2: Reduction reaction using borane dimethylsulfide ($BH_3S(CH_3)_2$)**

5 g (0.019 mol) of (R)-2-(2-methoxyphenyl)-2-((tetrahydro-2H-pyran-4-yl)oxy)acetic acid of Formula 2 prepared in Example 4 was added to 50 ml of tetrahydrofuran. Thereafter, the internal temperature of the reaction solution was cooled to 5 °C or less, and 7.6 mℓ (0.075 mol) of borane-dimethylsulfide was slowly added. After stirring for 1 hour while maintaining the reaction temperature at 5 °C or less, the reaction temperature was gradually raised and the reaction solution was stirred at room temperature for 1 hour. The reaction solution was cooled to 10 °C or less, 100 mL of sodium bicarbonate was added, and extracted three times using 30 ml of ethyl acetate respectively. The organic layer was washed with 100 ml of aqueous saturated sodium chloride solution, dried over anhydrous magnesium sulfate, and distilled under reduced pressure to obtain 4.74 g (100%) of the titled compound (Formula 1) as a solid phase.
Melting point: 97°C~102°C
GC/MS spectrum: m/z=252.10(M)
1H NMR (300 MHz, CDC13): 7.41 (dd, 1H), 7.26 (t, 1H), 7.24 (t, 1H), 6.86 (d, 1H), 5.06 (dd, 1H), 3.97-3.88 (m, 2H), 3.82 (s, 3H), 3.80-3.60 (m, 1H), 3.52-3.48 (m, 2H), 3.41-3.34 (m, 2H), 2.30-2.20 (m, 1H), 2.04-1.76 (m, 2H), 1.67-1.61 (m, 2H).

## Claims

1. A method for preparing a compound of Formula 1 below comprising a step of performing a reduction reaction on a compound of Formula 2 below to obtain the compound of Formula 1:

[Formula 1]

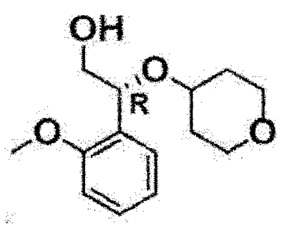

[Formula 2]

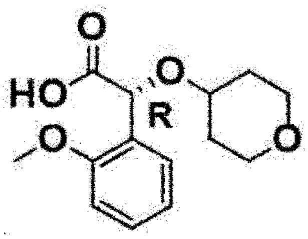
.

2. The method for preparing the compound of Formula 1 according to claim 1, wherein the reduction reaction is performed using at least one selected from the group consisting of lithium aluminum hydride ($LiAlH_4$, LAH), borane dimethyl sulfide ($BH_3S(CH_3)_2$,BMS), lithium borohydride ($LiBH_4$), aluminum hydride ($AlH_3$), and RED-AL (Vitride).

3. The method for preparing the compound of Formula 1 according to claim 1, wherein the compound of Formula 2 is prepared by comprising a step of neutralizing a compound of Formula 3 below in the presence of an acid, and then extracting to obtain the compound of Formula 2 from an organic layer:

[Formula 3]

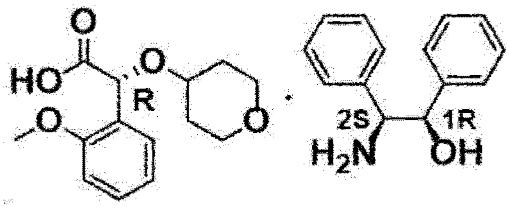
.

4. The method for preparing the compound of Formula 1 according to claim 3, wherein the neutralization of the compound of Formula 3 is carried out using at least one acid selected from the group consisting of hydrochloric acid, nitric acid, sulfuric acid, acetic acid, and trifluoroacetic acid.

5. The method for preparing the compound of Formula 1 according to claim 3, wherein the compound of Formula 3 is prepared by comprising a step of producing a salt from a compound of Formula 4 below and a compound of Formula 8 below:

[Formula 4]

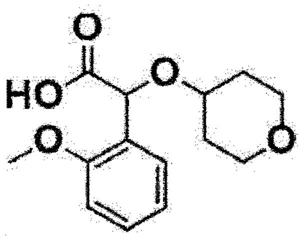

[Formula 8]

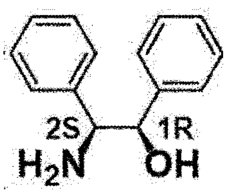
.

**6.** The method for preparing the compound of Formula 1 according to claim 5, wherein in the production of the salt, at least one solvent selected from the group consisting of methanol, ethanol, isopropanol, acetonitrile, toluene, acetone, methyl ethyl ketone, methyl isobutyl ketone, methyl acetate, ethyl acetate, isopropyl acetate, and diethyl ether is used.

**7.** The method for preparing the compound of Formula 1 according to claim 6, further comprising a step of adding the mixed solvent obtained by mixing water with the solvent used to form the salt to the already obtained salt compound, warming to the reflux temperature of the mixed solvent while stirring, and cooling it to room temperature and then purifying the reprecipitated solid by filtration, after producing the salt from the compound of Formula 4 and the compound of Formula 8.

**8.** The method for preparing the compound of Formula 1 according to claim 5, wherein the compound of Formula 4 is prepared by comprising a step of hydrolyzing a compound of Formula 5 below in the presence of a base:

[Formula 5]

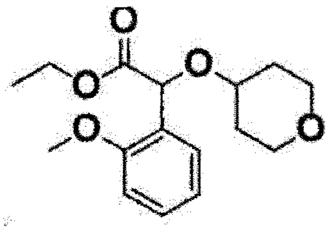

.

**9.** The method for preparing the compound of Formula 1 according to claim 8, wherein the hydrolysis is carried out using at least one base selected from the group consisting of potassium carbonate, calcium carbonate, sodium carbonate, potassium hydroxide, sodium hydroxide, lithium hydroxide, and sodium hydrogen carbonate.

**10.** The method for preparing the compound of Formula 1 according to claim 8, wherein the compound of Formula 5 is prepared by comprising a step of introducing a leaving group to a compound of Formula 6 below and performing a substitution reaction with a compound of Formula 7 below:

[Formula 6]

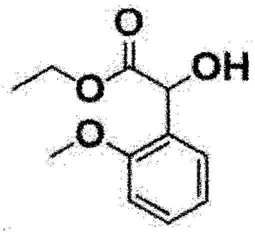

[Formula 7]

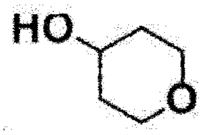

.

**11.** The method for preparing the compound of Formula 1 according to claim 10, wherein the leaving group is introduced by adding at least one selected from the group consisting of methanesulfonyl chloride, toluenesulfonyl chloride, benzenesulfonyl chloride, chlorine ($Cl_2$), bromine ($Br_2$), iodide ($I_2$), oxalyl chloride ($(COCl)_2$), thionyl chloride ($SOCl_2$), and sulfuryl chloride ($SO_2Cl_2$) to the reaction solution.

**12.** A compound represented by Formula 2 below which is an intermediate of the compound of Formula 1 according to claim 1:

[Formula 2]

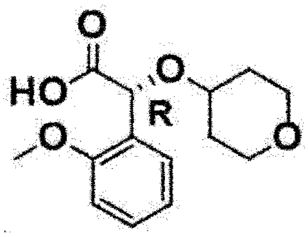

.

13. A compound represented by Formula 3 below which is an intermediate of the compound of Formula 1 according to claim 1:

[Formula 3]

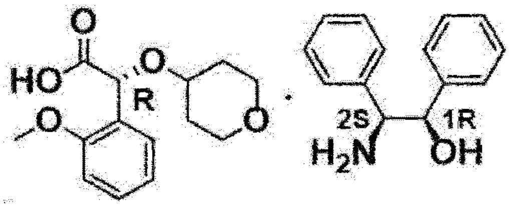

.

14. A compound represented by Formula 4 below which is an intermediate of the compound of Formula 1 according to claim 1:

[Formula 4]

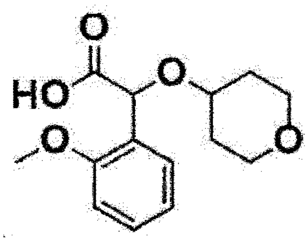

.

15. A compound represented by Formula 5 below which is an intermediate of the compound of Formula 1 according to claim 1:

[Formula 5]

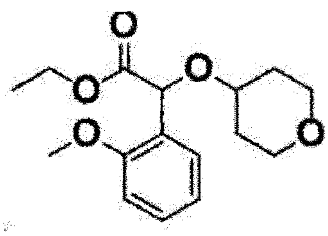

.

**INTERNATIONAL SEARCH REPORT**

International application No.
**PCT/KR2018/011383**

A. CLASSIFICATION OF SUBJECT MATTER

***C07D 309/10(2006.01)i, A61K 31/351(2006.01)i***

According to International Patent Classification (IPC) or to both national classification and IPC

B. FIELDS SEARCHED

Minimum documentation searched (classification system followed by classification symbols)
C07D 309/10; A61K 31/38; A61K 31/4412; A61K 31/519; C07C 201/16; C07C 205/15; C07D 309/12; C07D 495/04; A61K 31/351

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal), STN (Registry, Caplus, Casreact), Google & Keywords: 2R-2-(2-methoxyphenyl)-2-(oxane-4-yloxy) ethane-1-ol, intermediate body, reduction response

C. DOCUMENTS CONSIDERED TO BE RELEVANT

| Category* | Citation of document, with indication, where appropriate, of the relevant passages | Relevant to claim No. |
|---|---|---|
| Y<br>A | WO 2013-071169 A1 (NIMBUS APOLLO, INC.) 16 May 2013<br>See abstract; and paragraphs [00526]-[00528]. | 1-11<br>12-15 |
| Y | WO 2013-075083 A1 (CONSTELLATION PHARMACEUTICALS) 23 May 2013<br>See paragraphs [00526], [00527], [00611], [00612]. | 1-11 |
| Y | JP 2003-146943 A (DAIICHI FINE CHEMICAL CO., LTD.) 21 May 2003<br>See abstract; claims 1, 4; and paragraphs [0018], [0019], [0022], [0023], [0025]. | 3-11 |
| A | WO 2017-151816 A1 (GILEAD APOLLO, LLC.) 08 September 2017<br>See the entire document. | 1-15 |
| PX | WO 2018-161022 A1 (GILEAD SCIENCES, INC.) 07 September 2018<br>See paragraphs [0408]-[0413]. | 1,2,12,14,15 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 JANUARY 2019 (07.01.2019) | **07 JANUARY 2019 (07.01.2019)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

**INTERNATIONAL SEARCH REPORT**
Information on patent family members

International application No.
**PCT/KR2018/011383**

| Patent document cited in search report | Publication date | Patent family member | Publication date |
|---|---|---|---|
| WO 2013-071169 A1 | 16/05/2013 | AU 2012-335088 A1 | 29/05/2014 |
| | | AU 2012-335088 B2 | 24/08/2017 |
| | | AU 2017-265088 A1 | 14/12/2017 |
| | | BR 112014011351 A2 | 06/06/2017 |
| | | CA 2855372 A1 | 16/05/2013 |
| | | CL 2014001241 A1 | 28/11/2014 |
| | | CN 104105485 A | 15/10/2014 |
| | | CN 104105485 B | 12/04/2017 |
| | | CN 106905346 A | 30/06/2017 |
| | | DK 2776038 T3 | 23/04/2018 |
| | | EA 030264 B1 | 31/07/2018 |
| | | EA 201490826 A1 | 30/01/2015 |
| | | EA 201692104 A1 | 31/08/2017 |
| | | EP 2776038 A1 | 17/09/2014 |
| | | EP 2776038 B1 | 10/01/2018 |
| | | EP 3329919 A1 | 06/06/2018 |
| | | ES 2665580 T3 | 26/04/2018 |
| | | HK 1201207 A1 | 28/08/2015 |
| | | HR P20180534 T1 | 04/05/2018 |
| | | HU E037444 T2 | 28/09/2018 |
| | | JP 2014-533281 A | 11/12/2014 |
| | | JP 2017-114910 A | 29/06/2017 |
| | | JP 2018-052989 A | 05/04/2018 |
| | | JP 6114297 B2 | 12/04/2017 |
| | | JP 6276442 B2 | 07/02/2018 |
| | | KR 10-2014-0106554 A | 03/09/2014 |
| | | LT 2776038 T | 25/04/2018 |
| | | MX 2014005693 A | 26/11/2014 |
| | | MX 344490 B | 16/12/2016 |
| | | NZ 624819 A | 28/10/2016 |
| | | NZ 723933 A | 23/03/2018 |
| | | PL 2776038 T3 | 29/06/2018 |
| | | PT 2776038 T | 18/04/2018 |
| | | RS 57157 B1 | 31/07/2018 |
| | | SI 2776038 T1 | 29/06/2018 |
| | | TW 201350489 A | 16/12/2013 |
| | | TW 201400489 A | 01/01/2014 |
| | | TW 201720831 A | 16/06/2017 |
| | | TW I582095 B | 11/05/2017 |
| | | TW I612050 B | 21/01/2018 |
| | | UA 114901 C2 | 28/08/2017 |
| | | US 2013-0123231 A1 | 16/05/2013 |
| | | US 2015-0203510 A1 | 23/07/2015 |
| | | US 2016-0297834 A1 | 13/10/2016 |
| | | US 8969557 B2 | 03/03/2015 |
| | | US 9453026 B2 | 27/09/2016 |
| | | US 9944655 B2 | 17/04/2018 |
| WO 2013-075083 A1 | 23/05/2013 | EP 2780014 A1 | 24/09/2014 |

Form PCT/ISA/210 (patent family annex) (January 2015)

**INTERNATIONAL SEARCH REPORT**
Information on patent family members

International application No.
**PCT/KR2018/011383**

| Patent document cited in search report | Publication date | Patent family member | Publication date |
|---|---|---|---|
| | | EP 2780014 A4 | 01/07/2015 |
| | | US 2014-0288123 A1 | 25/09/2014 |
| | | US 9206128 B2 | 08/12/2015 |
| | | WO 2013-075083 A8 | 23/05/2013 |
| JP 2003-146943 A | 21/05/2003 | JP 4104319 B2 | 18/06/2008 |
| WO 2017-151816 A1 | 08/09/2017 | AR 107790 A1 | 06/06/2018 |
| | | AU 2017-226267 A1 | 06/09/2018 |
| | | CA 3015526 A1 | 08/09/2017 |
| | | CN 108699078 A | 23/10/2018 |
| | | KR 10-2018-0114937 A | 19/10/2018 |
| | | SG 11201807077 A | 27/09/2018 |
| | | TW 201738247 A | 01/11/2017 |
| | | US 2017-0267690 A1 | 21/09/2017 |
| WO 2018-161022 A1 | 07/09/2018 | US 2018-0298025 A1 | 18/10/2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013071169 A
- WO 2014182950 A
- WO 2014182951 A